# EUROPEAN PATENT APPLICATION

(11) **EP 4 603 506 A1**
(43) Date of publication of application: **20.08.2025**
(21) Application number: 23877747.8
(22) Date of filing: 13.10.2023
(51) Int. Cl.: C07K 14/605, A61K 38/26, A61P 3/08, A61K 38/16, A61K 38/17

(54) **NOVEL GLP-1 RECEPTOR ANTAGONIST, AND PHARMACEUTICAL COMPOSITION FOR PREVENTING OR TREATING CONGENITAL HYPERINSULINEMIA OR HYPOGLYCEMIA, COMPRISING SAME**

(30) Priority: 14.10.2022 KR 20220132605; 14.10.2022 KR 20220132606
(71) Applicant: Hanmi Pharm. Co., Ltd., Hwaseong-si, Gyeonggi-do 18536 (KR)
(72) Inventor: KIM, Eun Jung, Hwaseong-si, Gyeonggi-do 18469 (KR); PARK, Cho Rong, Hwaseong-si, Gyeonggi-do 18469 (KR); CHOI, Jae Hyuk, Hwaseong-si, Gyeonggi-do 18469 (KR); KIM, Won Ki, Hwaseong-si, Gyeonggi-do 18469 (KR); YE, Byeong Jin, Hwaseong-si, Gyeonggi-do 18469 (KR); IM, Hyeon Joo, Hwaseong-si, Gyeonggi-do 18469 (KR); SHIN, Min Kyung, Hwaseong-si, Gyeonggi-do 18469 (KR)
(74) Representative: Mewburn Ellis LLP
(86) International application number: PCT/KR2023/015820
(87) International publication number: WO 2024/080824

(57) **Abstract**

The present invention relates to a novel GLP-1 receptor antagonist analog and an acylated GLP-1 receptor analog, and use thereof for preventing or treating congenital hyperinsulinemia or hypoglycemia.

## Description

### [Technical Field]

The present invention relates to a pharmaceutical composition for preventing or treating congenital hyperinsulinemia or hypoglycemia containing a GLP-1 receptor antagonist analog or an acylated GLP-1 receptor antagonist analog conjugate.

### [Background Art]

Hypoglycemia refers to a condition where blood glucose levels are lower compared to those of normal individuals (60-120 mg/dL during fasting and 140 mg/dL or lower two hours after eating). A blood glucose level of 50 mg/dL or lower is typically determined to be hypoglycemic.

Hypoglycemia may occur due to the administration of excessive oral hypoglycemic agents or insulin, prolonged fasting, or excessive physical activity or exercise. Hypoglycemia may also occur due to factors such as severe physical diseases, deficiencies of hormones such as adrenal cortex hormones or glucagon, insulin-producing pancreatic tumors, and insulin autoimmune diseases, as well as in gastric resection patients and individuals with hereditary disorders of carbohydrate metabolism enzymes.

Meanwhile, congenital hyperinsulinism (CHI) is one of the most frequent causes of severe and persistent hypoglycemia occurring in newborns and children. Insulin, a hormone that regulates blood glucose in the human body, serves to reduce blood glucose levels when they increase due to food intake. However, patients with congenital hyperinsulinism fail to perform such a regulatory function and secrete insulin from the pancreas regardless of blood glucose levels. As a result, these patients experience hypoglycemia.

The occurrence of hypoglycemia or hypoglycemia due to congenital hyperinsulinism results in the failure to maintain glucose, ketone, and lactatesubstances primarily used by brain cells-and blocks the supply of energy from proteins or fats in the body. This causes brain cell damage, which can lead to seizures, learning disabilities, cerebral palsy, blindness, and, in severe cases, death.

Hypoglycemia may occur due to temporary excessive insulin secretion and may also occur in infants who have undergone fetal distress. The cause of abnormal insulin secretion is not clear, but in such cases, the condition improves within a few days to months. Hypoglycemia may also occur temporarily when blood glucose is not well-regulated in mothers with diabetes, but it does not reoccur once hypoglycemia resolves through appropriate breastfeeding. Another cause is persistent hyperinsulinism resulting from various genetic defects. Studies have reported that the causes of hyperinsulinism due to genetic defects include mutations in the SUR gene or Kir6.2 gene on chromosome 11p15.1, increased glucokinase (GK) activity due to mutations in the GK gene on chromosome 7p15-p13, and elevated ATP levels in pancreatic beta islet cells resulting from the activation of glutamate dehydrogenase (GDH) due to mutations in the GDH gene.

Bariatric surgeries performed to treat complications resulting from severe obesity are classified into laparoscopic adjustable gastric banding (LAGB), Roux-en-Y gastric bypass (RYGB), sleeve gastrectomy (SG), and biliopancreatic diversion (BPD). Hypoglycemia caused by dumping syndrome has been reported as a complication of bariatric surgery.

GLP-1 receptor antagonists are medications administered for the purpose of increasing appetite (KR 10-2001-0089563 A). Exendin-3(9-39) is a representative example thereof (Indraneel Banerjee, Mark J. Dunne, in Encyclopedia of Endocrine Diseases (Second Edition), 2019), and the administration of exendin-3(9-39) has been reported to suppress insulin secretion and exhibit therapeutic effects in acute hypoglycemia (Calabria AC, Li C, Gallagher PR, Stanley CA, De Leon DD. GLP-1 receptor antagonist exendin-(9-39) elevates fasting blood glucose levels in congenital hyperinsulinism owing to inactivating mutations in the ATP-sensitive K+ channel. Diabetes. 2012;61(10):2585-2591. doi:10.2337/db12-0166).

### [Disclosure]

### [Technical Problem]

Therapeutic agents for congenital hyperinsulinemia and hypoglycemia using GLP-1 receptor antagonists are still insufficiently developed, and thus the development of effective therapeutic agents is needed.

### [Technical Solution]

An aspect of the present invention is to provide a GLP-1 receptor antagonist analog or an acylated GLP-1 receptor antagonist analog conjugate.

Another aspect of the present invention is to provide use of the GLP-1 receptor antagonist analog, the acylated GLP-1 receptor antagonist analog conjugate, or a composition containing the analog or conjugate for preventing or treating congenital hyperinsulinemia or hypoglycemia.

Still another aspect of the present invention is to provide a pharmaceutical composition for preventing or treating congenital hyperinsulinemia or hypoglycemia containing the GLP-1 receptor antagonist analog or the acylated GLP-1 receptor antagonist analog conjugate.

Still another aspect of the present invention is to provide a method for preventing or treating congenital hyperinsulinemia or hypoglycemia, the method including administering the pharmaceutical composition to a subject in need thereof.

Still another aspect of the present invention is to provide use for providing a medication for preventing or treating congenital hyperinsulinemia or hypoglycemia containing the GLP-1 receptor antagonist analog, the acylated GLP-1 receptor antagonist analog conjugate, or the composition containing the analog or conjugate.

### [Advantageous Effects]

The GLP-1 receptor antagonist analog and the acylated GLP-1 receptor antagonist analog conjugate of the present invention can serve as an antagonist by acting on GLP-1 receptors, leading to an effect in the prevention or treatment of congenital hyperinsulinemia or hypoglycemia.

### [Brief Description of Drawings]

FIG. 1 shows the results of measuring blood glucose levels over time in the acylated GLP-1 receptor antagonist analog conjugate (SEQ ID NO: 17) according to the present invention.
FIG. 2 shows the results of measuring blood glucose levels in vertical sleeve gastrectomy rats by the administration of the acylated GLP-1 receptor antagonist analog conjugate (SEQ ID NO: 17) according to the present invention.
FIG. 3 shows the results of measuring plasma insulin levels in vertical sleeve gastrectomy rats by the administration of the acylated GLP-1 receptor antagonist analog conjugate (SEQ ID NO: 17) according to the present invention.

### [Detailed Description of the Invention]

An aspect of the present invention is directed to a novel GLP-1 receptor antagonist analog or acylated GLP-1 receptor antagonist analog.

In an embodiment, the GLP-1 receptor antagonist analog may be represented by General Formula 1 below:
X1-X2-X3-X4-X5-X6-X7-X8-X9-X10-E-E-X13-A-X15-R-X17-F-I-X20-W-L-X23-X24-G-G-P-S-S-G-A-P-P-P-S-X36 (General Formula 1, SEQ ID NO: 25) wherein, in General Formula 1,
X1 is threonine or absent;
X2 is phenylalanine or absent;
X3 is threonine or absent;
X4 is serine or absent;
X5 is aspartic acid or absent;
X6 is leucine, valine, or absent;
X7 is serine or absent;
X8 is alanine, lysine, serine, or absent;
X9 is glutamine or tyrosine;
X10 is methionine or leucine;
X13 is glutamic acid or glutamine;
X15 is alanine or valine;
X17 is leucine or glutamic acid;
X20 is glutamic acid or alanine;
X23 is lysine, valine, or an acylated amino acid;
X24 is asparagine, lysine, or an acylated amino acid;
X36 is cysteine, lysine, an acylated amino acid, or absent; and
the symbol "-" represents a peptide bond.

In another embodiment, with respect to the GLP-1 receptor antagonist analog,
in General Formula 1,
X5 is aspartic acid;
X6 is leucine or valine;
X7 is serine;
X8 is lysine or serine;
X15 is valine;
X23 is lysine or valine;
X24 is asparagine or lysine.

In still another embodiment, with respect to the GLP-1 receptor antagonist analog,
in General Formula 1,
X1 to X4 are absent;
X5 is aspartic acid;
X6 is leucine;
X7 is serine;
X8 is serine;
X9 is tyrosine;
X10 is leucine;
X13 is glutamic acid;
X15 is valine;
X17 is leucine;
X20 is glutamic acid;
X23 is lysine;
X24 is asparagine; and
X36 is lysine or an acylated amino acid.

In the GLP-1 receptor antagonist analog according to any one of the previous embodiments, the acylated amino acid may be any one of amino acids represented by K(1) to K(4):
[K(1)] C20diacid-γGlu-(AEEA)₂-Lys; 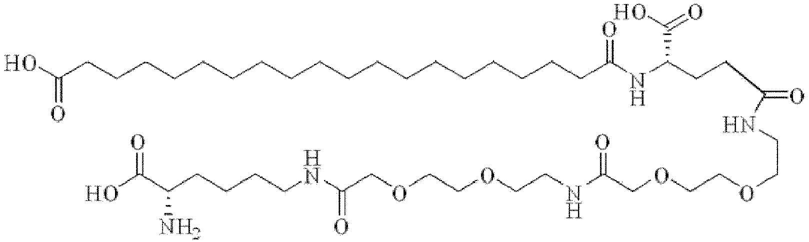
[K(2)] C18diacid-γGlu-(AEEA)₂-Lys; 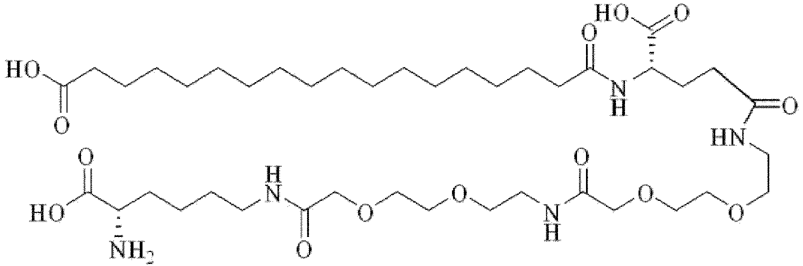
[K(3)] C16acid-γGlu-(AEEA)₂-Lys; and 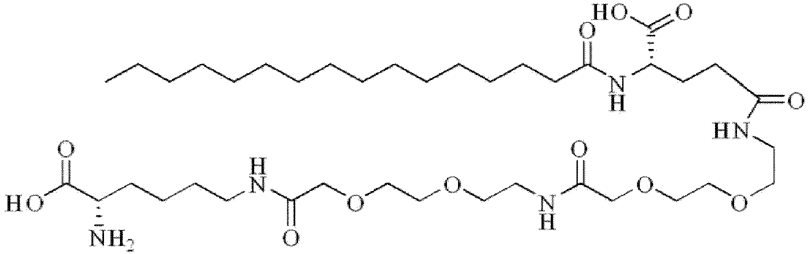
[K(4)] C16diacid-γGlu-(AEEA)₂-Lys.

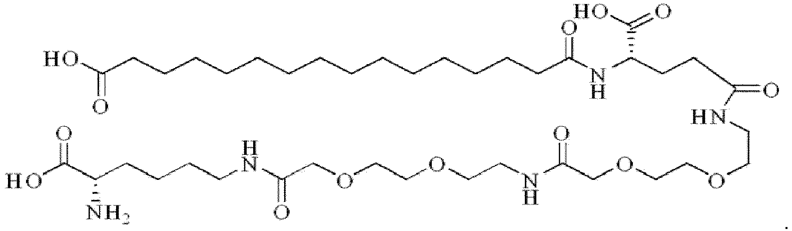

In the GLP-1 receptor antagonist analog according to any one of the previous embodiments, an acyl group may be attached to at least one amino acid of the GLP-1 receptor antagonist analog directly or via a linker.

In the GLP-1 receptor antagonist analog according to any one of the previous embodiments, the linker may contain (2-(2-aminoethoxy)ethoxy)acetic acid (AEEA).

In the GLP-1 receptor antagonist analog according to any one of the previous embodiments, the linker may be γ Glu-(AEEA)₂.

In the GLP-1 receptor antagonist analog according to any one of the previous embodiments, the linker may contain 0 to 3 AEEA molecules, and 0 to 3 gamma-glutamate molecules may be connected to the AEEA.

In the GLP-1 receptor antagonist analog according to any one of the previous embodiments, the C-terminus of the GLP-1 receptor antagonist analog may be amidated.

In the GLP-1 receptor antagonist analog according to any one of the previous embodiments, the GLP-1 receptor antagonist analog may be acylated with a C1 to C30 straight or branched chain acyl group containing one or two carboxylic acids.

In the GLP-1 receptor antagonist analog according to any one of the previous embodiments, the acyl group may be a C4 to C30 fatty acid or dicarboxylic acid.

In the GLP-1 receptor antagonist analog according to any one of the previous embodiments, an amino acid located at the C-terminus or lysine residue of the GLP-1 receptor antagonist analog may be acylated.

In the GLP-1 receptor antagonist analog according to any one of the previous embodiments, the C-terminus of the GLP-1 receptor antagonist analog may be amidated.

In the GLP-1 receptor antagonist analog according to any one of the previous embodiments, the GLP-1 receptor antagonist analog may include any one sequence selected from the group consisting of the amino acid sequences of SEQ ID NOs: 1 to 22.

In the GLP-1 receptor antagonist analog according to any one of the previous embodiments, the GLP-1 receptor antagonist analog may include any one sequence selected from the group consisting of the amino acid sequences of SEQ ID NOs: 3, 7 to 12, 21, and 22.

In the GLP-1 receptor antagonist analog according to any one of the previous embodiments, the GLP-1 receptor antagonist analog may include any one sequence selected from the group consisting of the amino acid sequences of SEQ ID NOs: 17, 19, 21, and 22.

In the GLP-1 receptor antagonist analog according to any one of the previous embodiments, the GLP-1 receptor antagonist analog may have any one structure of (i) to (iv) below:

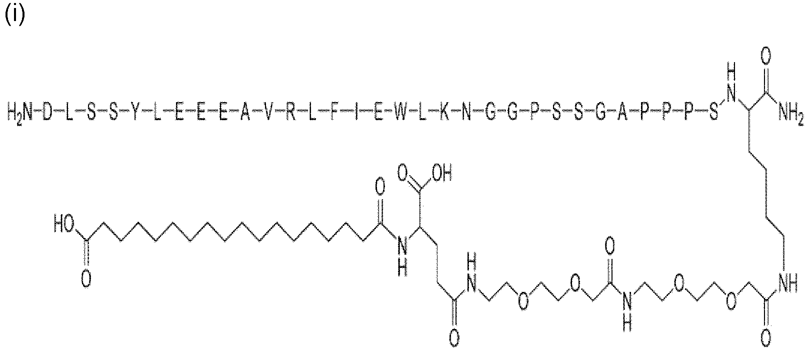

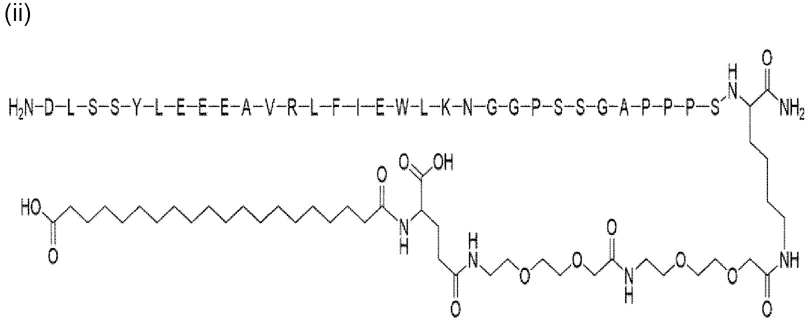

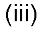

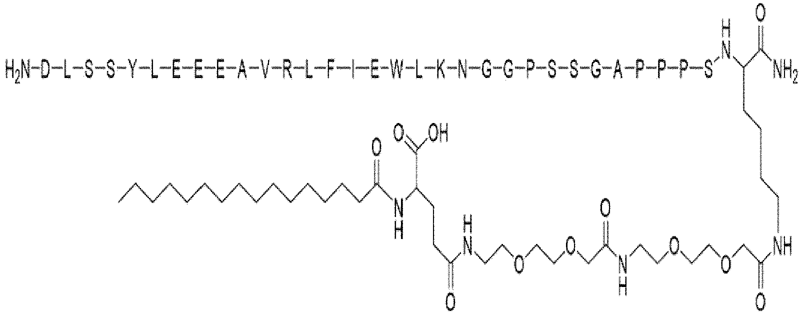

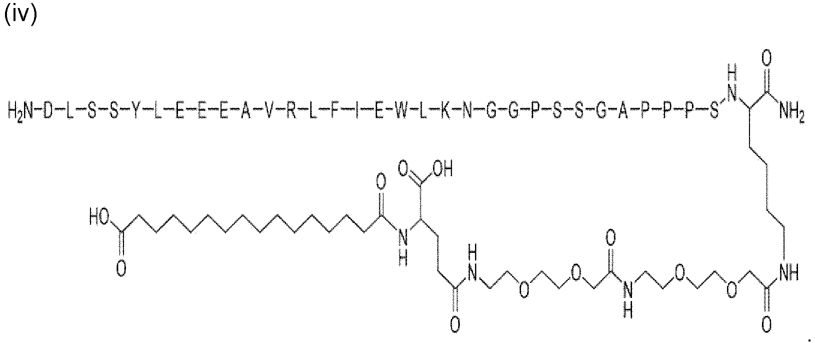

Another aspect of the present invention is directed to a pharmaceutical composition for preventing or treating congenital hyperinsulinemia or hypoglycemia, containing the GLP-1 receptor antagonist analog or acylated GLP-1 receptor antagonist analog in a pharmaceutically effective amount.

In an embodiment, the pharmaceutical composition may further contain a pharmaceutically acceptable carrier.

In another embodiment, the pharmaceutical composition may increase the blood glucose level in a subject when administered.

In the pharmaceutical composition according to any one of the previous embodiments, the pharmaceutical composition may reduce the secretion of insulin in a subject when administered.

In the pharmaceutical composition according to any one of the previous embodiments, the hypoglycemia may be postbariatric hypoglycemia (PBH).

In the GLP-1 receptor antagonist analog according to any one of the previous embodiments, the postbariatric hypoglycemia may be caused by bariatric surgery.

Still another aspect of the present invention is directed to use of the GLP-1 receptor antagonist analog, the acylated GLP-1 receptor antagonist analog, or the composition containing the same for preventing or treating congenital hyperinsulinemia or hypoglycemia.

Still another aspect of the present invention is directed to use of the GLP-1 receptor antagonist analog, the acylated GLP-1 receptor antagonist analog, or the composition containing the same in providing a medication for preventing or treating congenital hyperinsulinemia or hypoglycemia.

### [Mode for Carrying Out the Invention]

Hereinafter, detailed contents for implementing the present invention will be described below. Each description and embodiment disclosed in the present application may also be applied to other descriptions and embodiments. That is, all combinations of various elements disclosed in the present application fall within the scope of the present disclosure. Furthermore, the scope of the present invention is not limited by the specific description below.

Throughout the description herein, not only the typical one-letter and three-letter codes for naturally occurring amino acids, but also three-letter codes generally allowed for other amino acids, such as 2-aminoisobutyric acid (Aib), N-methylglycine (Sar), and α-methyl-glutamic acid, are used. The amino acids mentioned herein are abbreviated according to the nomenclature rules of IUPAC-IUB as follows.

| | | | |
|---|---|---|---|
| Alanine | A | Arginine | R |
| Asparagine | N | Aspartic acid | D |
| Cysteine | C | Glutamic acid | E |
| Glutamine | Q | Glycine | G |
| Histidine | H | Isoleucine | I |
| Leucine | L | Lysine | K |
| Methionine | M | Phenylalanine | F |
| Proline | P | Serine | S |
| Threonine | T | Tryptophan | W |
| Tyrosine | Y | Valine | V |

An aspect of the present invention is directed to a GLP-1 receptor antagonist analog or a conjugate where the GLP-1 receptor antagonist analog is acylated.

GLP-1, which is a hormone secreted in the small intestine stimulated by food intake, promotes insulin secretion in the pancreas and suppresses the secretion of glucagon, thus helping the action of lowering blood glucose concentrations. Additionally, GLP-1 serves to lower digestive action in the gastrointestinal tract by acting as a satiety factor and to reduce food intake by delaying the passage time of digested food through the gastrointestinal tract.

The blood glucose regulation and weight loss effects of GLP-1 are being used to develop therapeutic agents for diabetes and obesity, and exendin-4, a GLP-1 receptor agonist, is known as a representative example.

Native GLP-1 has the following sequence (SEQ ID NO: 23):
HAEGTFTSDVSSYLEGQAAKEFIAWLVKGRG

As used herein, the term "GLP-1 receptor antagonist" refers to a substance that acts on the GLP-1 receptor to suppress or weaken the activity of GLP-1. Exendin-3(9-39) is a known GLP-1 receptor antagonist (Montrose-Rafizaden et al., 1997, J. Biol. Chem. 272(34):21201-21206).

Exendin-3(9-39) has the following sequence (SEQ ID NO: 24):
DLSKQMEEEAVRLFIEWLKNGGPSSGAPPPS

As used herein, the term "GLP-1 receptor antagonist analog" or "analog" refers to a substance that antagonizes the GLP-1 receptor (GLP-1R). The GLP-1 receptor antagonist analog is, but not limited to, a peptide with at least one difference in the amino acid sequence compared with native GLP-1, and may be obtained by variation of the native GLP-1 sequence through any one variation of substitution, addition, deletion, and modification or a combination of thereof in a part of the sequence. The GLP-1 receptor antagonist analog may be non-naturally occurring.

Additionally, such a variation for the preparation of the GLP-1 receptor antagonist analog includes all of the following: variations using L-type or D-type amino acid(s) and/or non-native amino acid(s); and/or variations made by modification of the sequence of the native form, for example, a variation of a side chain functional group, an intramolecular covalent bonding (e.g., a ring formation between side chains), methylation, acylation, ubiquitination, phosphorylation, aminohexanation, biotinylation, and the like. The variation also includes substitution with a non-native compound.

The GLP-1 receptor antagonist analog may be obtained by adding one or more amino acids to the amino and/or carboxy terminus of the native GLP-1, but is not limited thereto.

The amino acids that are substituted or added may be not only 20 amino acids commonly found in human proteins but also atypical or non-naturally occurring amino acids. Commercial sources of atypical amino acids may include Sigma-Aldrich, ChemPep, and Genzyme Pharmaceuticals. The peptides including these amino acids and typical peptide sequences may be synthesized and purchased from companies specialized in the synthesis of commercial peptides, for example, the American Peptide Company and Bachem in the USA, or Anygen in Korea.

In accordance with one specific aspect, the GLP-1 receptor antagonist analog of the present invention may be a GLP-1 receptor antagonist analog represented by, but is not limited to, General Formula 1 below:
X1-X2-X3-X4-X5-X6-X7-X8-X9-X10-E-E-X13-A-X15-R-X17-F-I-X20-W-L-X23-X24-G-G-P-S-S-G-A-P-P-P-S-X36 (General Formula 1, SEQ ID NO: 25) wherein, in General Formula 1,
X1 is threonine or absent;
X2 is phenylalanine or absent;
X3 is threonine or absent;
X4 is serine or absent;
X5 is aspartic acid or absent;
X6 is leucine, valine, or absent;
X7 is serine or absent;
X8 is alanine, lysine, serine, or absent;
X9 is glutamine or tyrosine;
X10 is methionine or leucine;
X13 is glutamic acid or glutamine;
X15 is alanine or valine;
X17 is leucine or glutamic acid;
X20 is glutamic acid or alanine;
X23 is lysine, valine, or an acylated amino acid;
X24 is asparagine, lysine, or an acylated amino acid;
X36 is cysteine, lysine, an acylated amino acid, or absent; and
the symbol "-" represents a peptide bond.

In a specific embodiment, with respect to the GLP-1 receptor antagonist analog,
in General Formula 1,
X5 is aspartic acid;
X6 may be leucine or valine;
X7 may be serine;
X8 may be lysine or serine;
X15 may be valine;
X23 may be lysine or valine;
X24 may be asparagine or lysine, but is not limited thereto.

The GLP-1 receptor antagonist analog may include any one sequence selected from the group consisting of amino acid sequences of SEQ ID NOs: 1 to 3, 5, 7 to 12, and 17 to 22, and more specifically, any one sequence selected from the group consisting of amino acid sequences of SEQ ID NOs: 3, 7 to 12, 21, and 22, but is not limited thereto.

In another specific embodiment, with respect to the GLP-1 receptor antagonist analog,
in General Formula 1,
X1 to X4 may be absent;
X5 may be aspartic acid;
X6 may be leucine;
X7 may be serine;
X8 may be serine;
X9 may be tyrosine;
X10 may be leucine;
X13 may be glutamic acid;
X15 may be valine;
X17 may be leucine;
X20 may be glutamic acid;
X23 may be lysine;
X24 may be asparagine;
X36 may be lysine or an acylated amino acid, but is not limited thereto.

In another embodiment, the GLP-1 receptor antagonist analog may have, but is not limited to, any one structure of (i) to (iv) below:

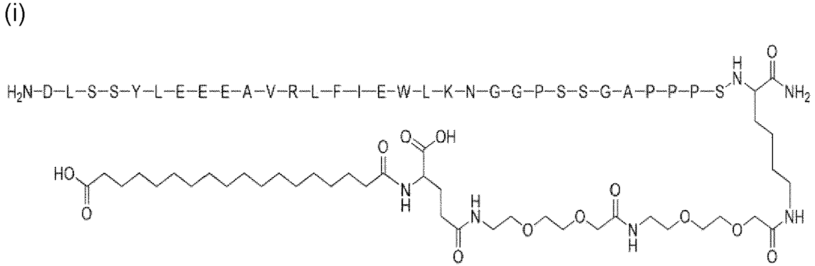

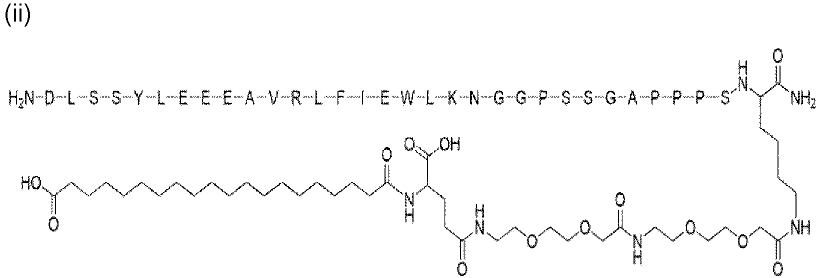

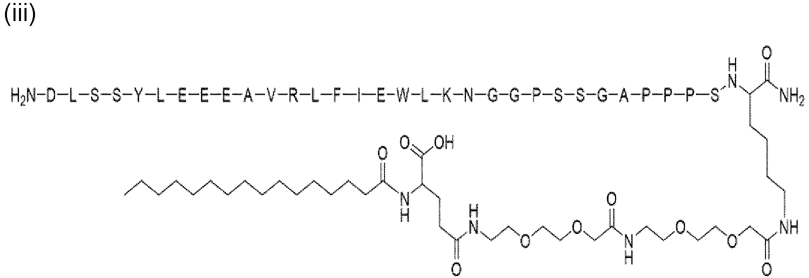

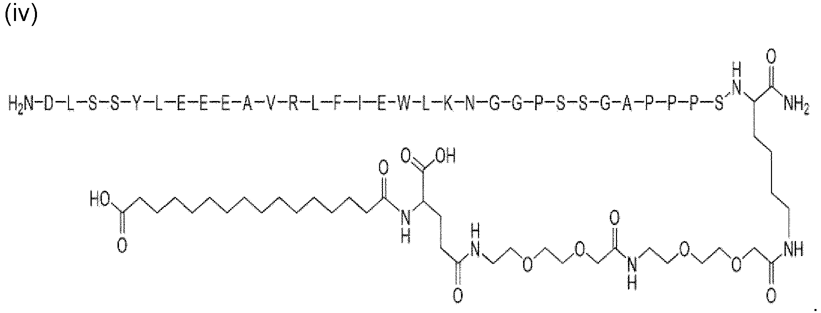

Alternatively, the GLP-1 receptor antagonist analog may include any one sequence selected from the group consisting of the amino acid sequences of SEQ ID NOs: 17, 19, 21, and 22, but is not limited thereto.

In an embodiment, the GLP-1 receptor antagonist analog including the amino acid sequence of SEQ ID NO: 17 may have the following structure, but is not limited thereto.

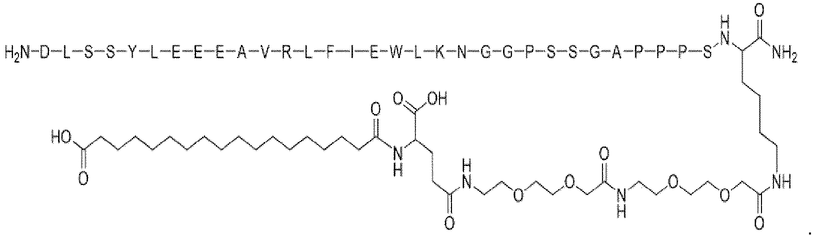

In an embodiment, the GLP-1 receptor antagonist analog including the amino acid sequence of SEQ ID NO: 19 may have the following structure, but is not limited thereto.

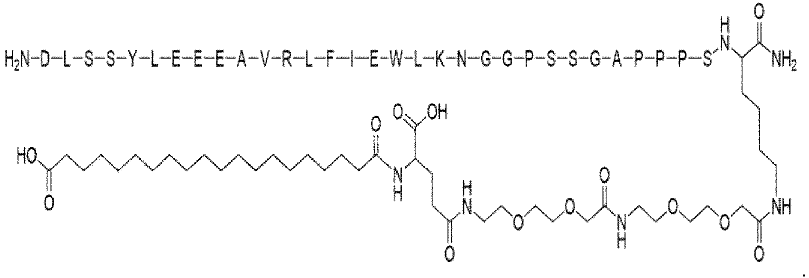

In an embodiment, the GLP-1 receptor antagonist analog including the amino acid sequence of SEQ ID NO: 21 may have the following structure, but is not limited thereto.

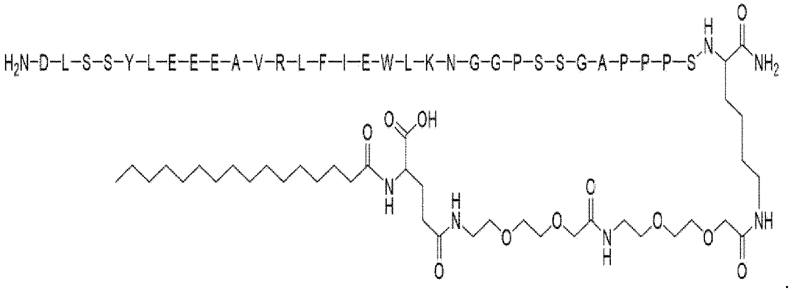

In an embodiment, the GLP-1 receptor antagonist analog including the amino acid sequence of SEQ ID NO: 22 may have the following structure, but is not limited thereto.

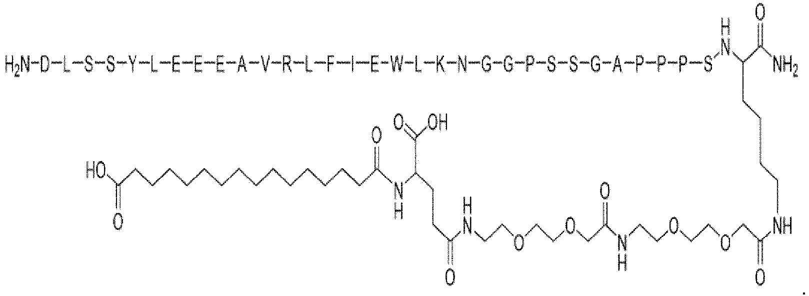

In the present invention, the GLP-1 receptor antagonist analog may be an acylated GLP-1 receptor antagonist analog conjugate with an attached acyl group, but is not limited thereto. In the present invention, the GLP-1 receptor antagonist analog may refer to both an acylated GLP-1 receptor antagonist analog and a nonacylated GLP-1 receptor antagonist analog.

The acylation is known as a method for improving the pharmacokinetic and pharmacodynamic properties of peptide drugs. Peptide drugs are problematic in terms of exhibiting efficacy due to enzyme degradation in the body. Therefore, enzyme action sites are blocked by peptide acylation through the attachment of a fatty acid to the peptide, thereby enhancing the stability of the peptide drugs and increasing the half-life of the peptide drugs. For the purpose of the present invention, the GLP-1 receptor antagonist analog of the present invention may be in an acylated from to increase the half-life.

As used herein, the term "acylated GLP-1 receptor antagonist analog conjugate" may be used interchangeably with "acylated GLP-1 receptor antagonist analog", "acylated analog", or "conjugate".

In the acylated GLP-1 receptor antagonist analog conjugate, an acyl group may be attached to an amino acid of the GLP-1 receptor antagonist analog via a linker, wherein the linker may include (2-(2-aminoethoxy)ethoxy)acetic acid (AEEA), and more specifically, may include the additional linkage of γ-glutamate, but is not limited thereto. A specific example of the linker may be (γGlu)ₘ-(AEEA)ₙ, wherein m and n each may be independently an integer of 0, 1, 2, 3, or greater, but is not limited thereto.

The acyl group may be linked to an amine group, hydroxyl group, thiol group, carboxyl group, or the like of an amino acid of the GLP-1 receptor antagonist analog through an amine group, hydroxyl group, thiol group, or the like of the linker, but the linker is not limited to a specific type or length as long as the acyl group can be linked to the GLP-1 receptor antagonist analog to contribute to the structural stability and the increase in the half-life. In addition, the linker may be covalently linked to the acyl group, and may be linked to the acyl group in one, two, three, or more repetitions, but is not limited thereto.

The acyl group used in the acylation may have a carbon chain with any length, or may have a linear or branched chain. Specifically, examples of the chain may include a linear aliphatic chain, a branched aliphatic chain, a chain containing a cyclic alkyl moiety, a hydrophobic natural product, such as a steroid, an aralkyl chain, or an alkyl chain containing an acyl moiety.

In a specific embodiment, the acylated GLP-1 receptor antagonist analog may be acylated with a C1 to C30 straight or branched chain acyl group containing one or more, specifically, one or two carboxylic acids. As one example, the acyl group may be a fatty acid or dicarboxylic acid, specifically, a C4 to C30 fatty acid or dicarboxylic acid, but is not limited thereto. As a more specific example, the acyl group may be a C16, C18, C20, C22, C24, C26, C28, or C30 fatty acid or dicarboxylic acid. Other examples of the acyl group may include bile acids, such as cholic acid, chenodeoxycholic acid, deoxycholic acid, lithocholic acid, taurocholic acid, glycocholic acid, and cholesteric acid, succinic acid or succinic acid derivatives, maleic acid or maleic acid derivatives, and the like.

The acylated GLP-1 receptor antagonist analog of the present invention may be obtained by attaching an acyl group to a GLP-1 receptor antagonist analog by a method known in the art, or may be prepared by synthesizing a peptide using an acylated amino acid, but is not limited thereto.

In the present invention, the acylated GLP-1 receptor antagonist analog may be in the form in which an acyl group is directly attached to an amino acid residue of a GLP-1 receptor antagonist analog. For example, the attachment of the acyl group may be made through an ester, thioester, or amide linkage, but is not limited thereto. Specifically, in the acylated GLP-1 receptor antagonist analog, an amino acid residue having an amine, hydroxyl, or thiol group may be acylated. For example, in the acylated GLP-1 receptor antagonist analog, an amino acid located at the C-terminus or lysine residue may be acylated.

In the present invention, the acylated amino acid of the acylated GLP-1 receptor antagonist analog may include, but is not limited thereto, any one of amino acid represented by K(1) to K(4) below:
[K(1)] C20diacid-γGlu-(AEEA)₂-Lys 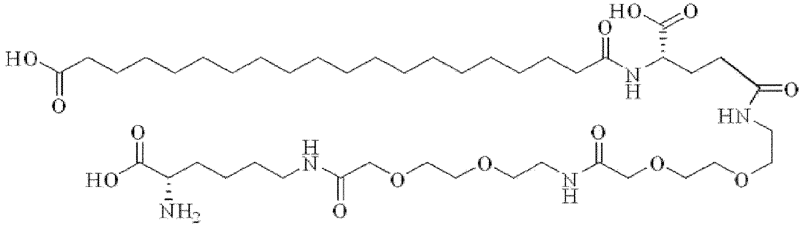
[K(2)] C18diacid-γGlu-(AEEA)₂-Lys 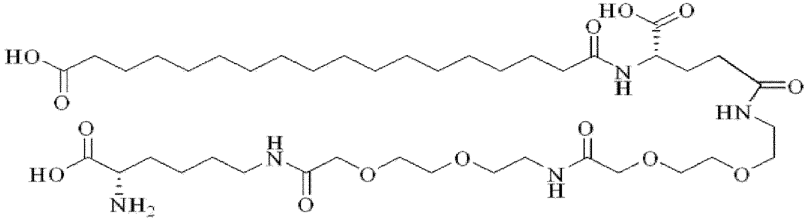
[K(3)] C16acid-γGlu-(AEEA)₂-Lys 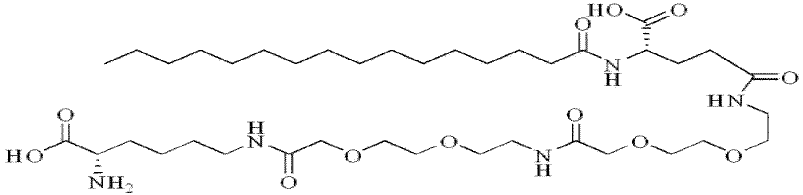
[K(4)] C16diacid-γGlu-(AEEA)₂-Lys

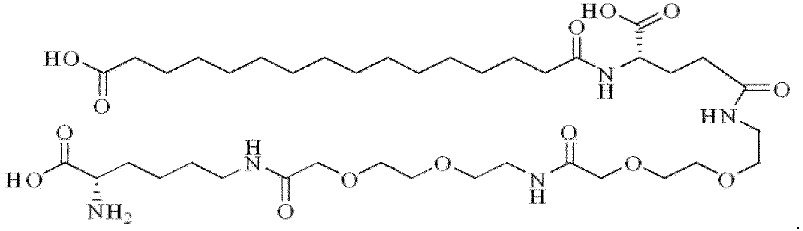

The acylated GLP-1 receptor antagonist analog conjugate of the present invention may contain an acylated amino acid at position 8, 23, 24, or 36, but is not limited thereto.

In accordance with a specific aspect, the GLP-1 receptor antagonist analog according to the present invention may include any one sequence among the amino acid sequences of SEQ ID NOs 1 to 22, and specifically, may consist (essentially) of an amino acid sequence selected from the group consisting of SEQ ID NOs: 1 to 22, but is not limited thereto.

Specifically, the GLP-1 receptor antagonist analog may include any one sequence among amino acid sequences of SEQ ID NOs 1 to 12, and specifically, may consist (essentially) of an amino acid sequence selected from the group consisting of SEQ ID NOs: 1 to 12, but is not limited thereto.

More specifically, the GLP-1 receptor antagonist analog may include any one sequence selected from the group consisting of amino acid sequences of SEQ ID NOs: 3, 7 to 12, 21, and 22, and specifically, may consist (essentially) of an amino acid sequence selected from the group consisting of SEQ ID NOs: 3, 7 to 12, 21, and 22, but is not limited thereto.

More specifically, the GLP-1 receptor antagonist analog may include any one sequence selected from the group consisting of amino acid sequences of SEQ ID NOs: 17, 19, 21, and 22, and specifically, may consist (essentially) of an amino acid sequence selected from the group consisting of SEQ ID NOs: 17, 19, 21, and 22, but is not limited thereto.

In accordance with another specific aspect, the acylated GLP-1 receptor antagonist analog conjugate according to the present invention may include any one sequence among SEQ ID NOs: 13 to 22, specifically, may consist (essentially) of an amino acid sequence selected from the group consisting of SEQ ID NOs: 13 to 22, but is not limited thereto.

Although described as a "peptide consisting of a specific sequence number" herein, this wording does not exclude the addition of a nonsense sequence, a naturally-occurring mutation, or a silent mutation thereof upstream or downstream of the amino acid sequence of the corresponding sequence number as long as the peptide has the equivalent or corresponding activity to the peptide consisting of the amino acid sequence of the corresponding sequence number, and it is apparent that a peptide having such a sequence addition or mutation falls within the scope of the present invention.

Additionally, the GLP-1 receptor antagonist analog of the present invention may include an amino acid sequence having homology or identity of at least 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% with the amino acid sequences of SEQ ID NOs: 1 to 22, but is not limited thereto as long as the analog can act on the GLP-1 receptor to antagonize the GLP-1 receptor.

The term "homology" or "identity" as used herein refers to a degree of relevance between two given amino acid sequences or nucleotide sequences, and the term may be expressed as a percentage.

The sequence homology or identity of conserved polynucleotides may be determined by standard alignment algorithms, and default gap penalties established by the program to be used may be used together. Substantially, homologous or identical sequences may hybridize under moderately or highly stringent conditions along their entire sequence or a part thereof. It would be obvious that the hybridization also includes hybridization with a polynucleotide containing usual codons or codons considering codon degeneracy in the polynucleotide.

The terms homology and identity may be often used interchangeably with each other.

The homology, similarity, or identity between any two peptide sequences may be determined by, for example, a known computer algorithm, such as the "FASTA" program, by using default parameters as in Pearson et al (1988) [Proc. Natl. Acad. Sci. USA 85]:2444. Alternatively, these may be determined using the Needleman-Wunsch algorithm (Needleman and Wunsch, 1970, J. Mol. Biol. 48: 443-453), which is performed in the Needleman program of the European Molecular Biology Open Software Suite (EMBOSS) package (Rice et al., 2000, Trends Genet. 16: 276-277) (version 5.0.0 or versions thereafter) (including the GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.] (1988) SIAM J Applied Math 48: 1073). For example, the homology, similarity, or identity may be determined using BLAST of the National Center for Biotechnology Information database, or ClustalW.

The homology, similarity, or identity between peptides may be determined by comparing sequence information using the GAP computer program, for example, Needleman et al., (1970), J Mol Biol. 48:443, as known in Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. The default parameters for the GAP program may include: (1) a unary comparison matrix (containing a value of 1 for identities and 0 for non-identities) and the weighted comparison matrix (or EDNAFULL (EMBOSS version of NCBI NUC4.4) substitution matrix) of Gribskov et al. (1986) Nucl. Acids Res. 14:6745, as disclosed by Schwartz and Dayhoff, eds., Atlas of Protein Sequence and Structure, National Biomedical Research Foundation, pp. 353-358 (1979); (2) a penalty of 3.0 for each gap and an additional 0.10 penalty for each symbol in each gap (or gap open penalty 10, gap extension penalty 0.5); and (3) no penalty for end gaps. Therefore, the term "homology" or "identity" as used herein represents the relevance between sequences.

The GLP-1 receptor antagonist analog or acylated GLP-1 receptor antagonist analog conjugate of the present invention may exhibit activity of about 1% or more, 10% or more, 30% or more, 50% or more, 70% or more, 80% or more, 90% or more, 100% or more, 150% or more, 200% or more, 250% or more, 300% or more, or 350% or more, compared with the activity (100%) of exendin-3(9-39), which is a known GLP-1 receptor antagonist.

The term "about" as used herein refers to a range encompassing ±0.5, ±0.4, ±0.3, ±0.2, ±0.1, and the like, and includes all values in the range equivalent or similar to those stated after this term, but is not limited thereto.

The activity of the GLP-1 receptor antagonist analog or acylated GLP-1 receptor antagonist analog conjugate may be measured by a method known in the art, and the measurement is not limited to a particular method. For instance, the antagonism of the GLP-1 receptor antagonist analog or acylated GLP-1 receptor antagonist analog of the present invention may be analyzed by measuring the concentration of cAMP, of which the production is suppressed by a GLP-1 receptor antagonist analog.

Additionally, the GLP-1 receptor antagonist analog or acylated GLP-1 receptor antagonist analog conjugate according to the present invention may be in the form in which the N-terminus and/or C-terminus of the peptide is not modified. However, those variants where the N-terminus and/or C-terminus of the peptide is chemically modified or protected by organic groups, or amino acids are added to a terminus of the peptide for its protection from proteases *in vivo* while increasing its stability, may also fall within the scope of the present invention.

Particularly, a chemically synthesized peptide has electrically charged N- and C-termini, and thus for the elimination of these charges, the N-terminus may be acetylated and/or the C-terminus may be amidated, but is not particularly limited thereto.

In the GLP-1 receptor antagonist analog or acylated GLP-1 receptor antagonist analog conjugate according to the present invention, the C-terminus may be amidated, or the C-terminus may be both amidated and acylated, but is not limited thereto.

Unless specified otherwise herein, the detailed description or claims with respect to the GLP-1 receptor antagonist analog or acylated GLP-1 receptor antagonist analog conjugate according to the present invention may also be applied to the ranges including not only the corresponding analog or conjugate but also salts of the corresponding analog or conjugate (e.g., pharmaceutically acceptable salts of the peptide) or solvates thereof. Therefore, the corresponding description herein are also applied to a specific salt thereof, a specific solvate thereof, and a specific solvate of the specific salt thereof. These salts may be, for example, in the form in which any pharmaceutically acceptable salt is used. The type of salts is not particularly limited. However, the salts are preferably in the form that is safe and effective to a subject, for example, a mammal, but is not particularly limited thereto.

The term "pharmaceutically acceptable" refers to a substance that can be effectively used for a desired purpose without causing excessive toxicity, irritation, allergic reactions, and the like, within the scope of medical and pharmaceutical decision.

As used herein, the term "pharmaceutically acceptable salt" refers to a salt derived from pharmaceutically acceptable inorganic acids, organic acids, or bases. Suitable examples of the acids may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzene sulfonic acid, and the like. Suitable examples of the salt derived from the bases may include alkali metals, such as sodium and potassium, alkali earth metals, such as magnesium, ammonium, and the like.

As used herein, the term "solvate" refers to a complex formed of the peptide, conjugate, or salt thereof according to the present invention and a solvent molecule.

The analog of the present invention may be synthesized by a method well known in the art, for example, an automatic peptide synthesizer, according to the length thereof, or may be produced by genetic engineering technology.

Specifically, the analog of the present invention may be prepared by a standard synthesis method, a recombinant expression system, or any other method known in the art. Therefore, the GLP-1 receptor antagonist analog or acylated GLP-1 receptor antagonist analog conjugate according to the present invention may be synthesized by multiple methods including the following methods.
(a) a method where a peptide is synthesized by a solid-phase or liquidphase process stepwise or by fragment assembly and a final peptide product is separated and purified;
(b) a method where a nucleic acid construct encoding a peptide is expressed in a host cell and an expression product is collected from a host cell culture;
(c) a method where the *in vitro* cell-free expression of a nucleic acid construct encoding a peptide is performed and an expression product is collected therefrom; or
a method where peptide fragments are obtained by any combination of (a), (b), and (c), the fragments are linked to obtain a peptide, and then the corresponding peptide is collected.

The above description can be applied to other embodiments or aspects of the present invention, but is not limited thereto.

Another aspect of the present invention provides a composition containing the GLP-1 receptor antagonist analog or acylated GLP-1 receptor antagonist analog conjugate according to the present invention.

The GLP-1 receptor antagonist analog or acylated GLP-1 receptor antagonist analog conjugate is as described above.

Specifically, the composition may be a pharmaceutical composition, and more specifically, the composition may be used for preventing or treating congenital hyperinsulinemia or hypoglycemia.

A specific aspect of the present invention is directed to a pharmaceutical composition for preventing or treating congenital hyperinsulinemia or hypoglycemia, containing the GLP-1 receptor antagonist analog or acylated GLP-1 receptor antagonist analog conjugate at a pharmaceutically effective amount.

The containing of the GLP-1 receptor antagonist analog or acylated GLP-1 receptor antagonist analog conjugate at a pharmaceutically effective amount may mean a degree at which targeted pharmaceutical activity (e.g., prevention, alleviation, or treatment of congenital hyperinsulinemia or hypoglycemia) can be obtained, or a level at which toxicity or side effects are absent or slightly present, up to a pharmaceutically acceptable level, in a subject to be administered, but is not limited thereto. The pharmaceutically effective amount may be determined by comprehensively considering the number of times of administration, patient, formulation, and the like.

As used herein, the term "prevention" refers to any action that suppresses or delays the development of a target disease, for example, congenital hyperinsulinemia or hypoglycemia, by the administration of the GLP-1 receptor antagonist analog or acylated GLP-1 receptor antagonist analog conjugate, or the composition containing the analog or conjugate, while the term "treatment" refers to any action that alleviates or advantageously changes the symptoms of a target disease, for example, congenital hyperinsulinemia or hypoglycemia, by the administration of the GLP-1 receptor antagonist analog or acylated GLP-1 receptor antagonist analog conjugate, or the composition containing the analog or conjugate.

As used herein, the term "administration" refers to the introduction of a predetermined substance into a patient by any appropriate method, and the route of administration of the composition may include, but is not particularly limited to, any typical route, through which the composition can arrive at an *in vivo* target, and for example, the route of administration may be intraperitoneal administration, intravenous administration, intramuscular administration, subcutaneous administration, intradermal administration, oral administration, topical administration, intranasal administration, intrapulmonary administration, intrarectal administration, or the like.

The congenital hyperinsulinemia of the present invention is one of the causative diseases that induce severe and persistent hypoglycemia in newborns and children. The congenital hyperinsulinemia may be caused by temporary increases in insulin secretion in low birth weight infants or infants of diabetic mothers, dysfunctions of pancreatic cells due to genetic mutations, and the like.

The hypoglycemia of the present invention refers to a condition which the blood glucose level is lower than those of normal persons. The term typically refers to a condition in which the blood glucose level is 50 mg/dL or less, but is not particularly limited thereto. In the present invention, the hypoglycemia encompasses both acute hypoglycemia and chronic hypoglycemia.

The symptoms of hypoglycemia include lack of energy, body trembling, paleness, cold sweat, dizziness, agitation, anxiety, heart palpitations, hunger, headache, fatigue, and the like. If hypoglycemia persists for a long time, it may induce convulsion or seizure and cause shock, potentially resulting in fainting.

Specifically, the hypoglycemia in the present invention may be postbariatric hypoglycemia (PBH), and more specifically, hypoglycemia caused by bariatric surgery, but is not limited thereto.

The "postbariatric hypoglycemia" or "hypoglycemia caused by bariatric surgery" refers to hypoglycemia underwent by patients after bariatric surgery, such as sleeve gastrectomy, duodenal switch, and Roux-en-Y gastric bypass, and may also be caused by dumping syndrome, one of complications.

There is currently no clearly established treatment for post-bariatric hypoglycemia or hypoglycemia caused by bariatric surgery. All researchers agree that dietary and lifestyle modifications are the most important treatment methods. The principles of dietary therapy include reducing the amount of food consumed at once and chewing high-protein, high-fat, low-carbohydrate, and low-liquid meals in small portions 5-6 times. If hypoglycemia, abdominal pain, or convulsion occurs due to dumping syndrome, the symptoms may be managed by the administration of drugs, such as a sedative, an anticonvulsant, a glucose injection, a sedative, or an autonomic nervous system blocker.

The GLP-1 receptor antagonist analog or acylated GLP-1 receptor antagonist analog conjugate of the present invention antagonizes GLP-1 receptor, and thus can increase blood glucose levels in the body and/or reduce the secretion of insulin, thereby exhibiting preventive or therapeutic effects against congenital hyperinsulinemia or hypoglycemia, but is not limited thereto.

The pharmaceutical composition of the present invention may further contain a pharmaceutically acceptable carrier, excipient, or diluent. As used herein, the term "pharmaceutically acceptable" refers to a feature of having a sufficient amount to exhibit a therapeutic effect and not causing adverse effects, and can be easily determined by a skilled person in the art based on factors well known in the medical field, such as the type of disease, patient's age, body weight, health condition, and sex, drug sensitivity of a patient, route of administration, method of administration, frequency of administration, duration of treatment, drugs used in combination or concurrently.

The pharmaceutical composition containing the GLP-1 receptor antagonist analog or acylated GLP-1 receptor antagonist analog conjugate of the present invention may further include a pharmaceutically acceptable carrier. With respect to the carrier, although not particularly limited, a binder, a lubricant, a disintegrant, a solubilizer, a dispersant, a stabilizer, a suspender, a colorant, a flavoring agent, and the like may be used for oral administration, and a buffer, a preservative, an analgesic, a solubilizer, an isotonic agent, a stabilizer, and the like may be mixed for an injection. Additionally, a base, an excipient, a lubricant, a preservative, and the like may be used for topical administration.

The formulation of the composition of the present invention may be prepared in various manners by mixing with the above-described pharmaceutically acceptable carriers. For example, for oral administration, the composition of the present invention may be formulated in the form of a tablet, a troche, a capsule, an elixir, a suspension, a syrup, a wafer, or the like, and for the injection, the composition may be formulated as a single dose ampoule or a multi-dose form. The composition may be formulated into a solution, a suspension, a tablet, pills, a capsule, a sustained-release preparation, or the like.

Meanwhile, examples of the carrier, excipient, and diluent suitable for the preparation may include lactose, dextrose, sucrose, sorbitol, mannitol, xylitol, erythritol, maltitol, starch, acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, a mineral oil, and the like. The composition may further contain a filler, an anticoagulant, a lubricant, a humectant, a flavoring agent, a preservative, and the like.

Additionally, the pharmaceutical composition of the present invention may have any one formulation selected from the group consisting of a tablet, a pill, a powder, granules, a capsule, a suspension, an oral liquid preparation, an emulsion, a syrup, a sterile aqueous solution, a non-aqueous solvent, a lyophilized preparation, and a suppository.

The composition may be formulated into a preparation for unit dosage form suitable for the administration into a patient's body according to a conventional method in the pharmaceutical field, and may specifically be formulated into a preparation useful for the administration of peptide pharmaceuticals and administered by an oral administration route or a parenteral administration route including a dermal, intravenous, intramuscular, intraarterial, intramedullary, intrathecal, intraventricular, pulmonary, transdermal, subcutaneous, intraperitoneal, intranasal, gastrointestinal, topical, sublingual, intravaginal, or rectal route by way of an administration method commonly used in the art, but the formulation method and administration method are not limited thereto.

The GLP-1 receptor antagonist analog or acylated GLP-1 receptor antagonist analog conjugate of the present invention may be used by mixing with various pharmaceutically acceptable carriers, such as physiological saline or organic solvents, and in order to increase the stability or absorption, a carbohydrate such as glucose, sucrose or dextran, an antioxidant such as ascorbic acid or glutathione, a chelating agent, a low-molecular-weight protein, or other stabilizers may be used as a medication.

The dose and number of administrations of the pharmaceutical composition of the present invention are determined according to the type of drug as an active ingredient, together with several related factors such as the disease to be treated, administration route, patient's age, sex and weight, and severity of disease.

Although not particularly limited, the pharmaceutical composition of the present invention may contain the ingredient (active ingredient) in an amount of 0.01 to 99 %(w/v).

The total effective amount of the composition of the present invention may be administered to a patient in a single dose, or may be administered in multiple doses by a fractionated treatment protocol for a long period of time. In the pharmaceutical composition of the present invention, the content of the active ingredient may vary depending on the severity of a disease. Specifically, the preferred total dose of the GLP-1 receptor antagonist analog or acylated GLP-1 receptor antagonist analog conjugate thereof of the present invention may be about 0.3 to 0.9 mg per 1 kg of body weight of a patient per day. The dose may be determined based on the mass value of the GLP-1 receptor antagonist analog, or the mass value of the GLP-1 receptor antagonist analog in the acylated GLP-1 receptor antagonist analog conjugate, excluding the fatty acid moieties, that is, the summed mass value of only polypeptide moieties. However, with respect to the dose of the GLP-1 receptor antagonist analog or the acylated GLP-1 receptor antagonist analog conjugate, the effective dose for a patient is determined by considering various factors including patient's age, body weight, health condition, and sex, the severity of disease, diet, and rate of excretion, as well as the route of administration of the pharmaceutical composition and the number of times of treatment, and thus, considering these, a person skilled in the art would be able to determine an appropriate effective dose according to the particular use of the composition of the present invention. The pharmaceutical composition according to the present invention is not particularly limited to the formulation, administration route, and administration method thereof as long as the pharmaceutical composition can exhibit the advantageous effects of the present invention.

The pharmaceutical composition of the present invention can exhibit excellent *in vivo* efficacy duration and potency and allow for a reduced number and frequency of administration compared with other medications, but is not particularly limited thereto.

Still another aspect of the present invention provides a method for preventing or treating congenital hyperinsulinemia or hypoglycemia, the method including administering to a subject the GLP-1 receptor antagonist analog, the acylated GLP-1 receptor antagonist analog conjugate, or the composition containing the analog or conjugate.

The GLP-1 receptor antagonist analog or acylated GLP-1 receptor antagonist analog conjugate, the composition containing the same, congenital hyperinsulinemia, hypoglycemia, prevention, and treatment are as described above.

The subject herein refers to a subject suspected of having congenital hyperinsulinemia or hypoglycemia, wherein the subject suspected of having congenital hyperinsulinemia or hypoglycemia indicates a mammal, including humans, rats, livestock, and the like, having or being at risk of developing the corresponding disease, but any subject is included without limitation as long as the subject can be treated with the GLP-1 receptor antagonist analog or acylated GLP-1 receptor antagonist analog conjugate, or the composition containing the same. The pharmaceutical composition containing the GLP-1 receptor antagonist analog or acylated GLP-1 receptor antagonist analog conjugate of the present invention may be administered to a subject suspected of having congenital hyperinsulinemia or hypoglycemia, and thus can efficiently treat the subject. The congenital hyperinsulinemia or hypoglycemia are as described above.

The method of the present invention may include administering a pharmaceutically effective amount of the pharmaceutical composition containing the peptide. The appropriate total daily dose may be determined by a practitioner within accurate medical judgment, and may be administered once or several times in divided doses. For the purpose of the present invention, the specific therapeutically effective dose for a specific patient may be preferably applied differently depending on various factors well known in the medical art, including the type and degree of responses to be achieved, a specific composition including whether other preparations are occasionally used therewith, the patient's age, body weight, general health condition, sex and a diet, the time of administration, the route of administration, the secretion rate of the composition, the duration of treatment, other drugs used in combination or concurrently with the composition of the present invention, and similar factors well-known in the medical art.

In the method of the present invention, the GLP-1 receptor antagonist analog or acylated GLP-1 receptor antagonist analog conjugate, or the composition containing the same may be administered through a typical route to reach an *in vivo* target, and examples of the route of administration may include intraperitoneal administration, intravenous administration, intramuscular administration, subcutaneous administration, intradermal administration, oral administration, topical administration, intranasal administration, intrapulmonary administration, intrarectal administration, and the like.

Still another aspect of the present invention provides use of the GLP-1 receptor antagonist analog, the acylated GLP-1 receptor antagonist analog conjugate, or the composition for preventing or treating congenital hyperinsulinemia or hypoglycemia.

The GLP-1 receptor antagonist analog, the acylated GLP-1 receptor antagonist analog conjugate, and the composition, congenital hyperinsulinemia, and hypoglycemia are as described above.

Still another aspect of the present invention provides use of the GLP-1 receptor antagonist analog, the acylated GLP-1 receptor antagonist analog conjugate, or the composition in the preparation of a medication (or a pharmaceutical composition) for preventing or treating congenital hyperinsulinemia or hypoglycemia.

The GLP-1 receptor antagonist analog, the acylated GLP-1 receptor antagonist analog conjugate, and the composition, congenital hyperinsulinemia, and hypoglycemia are as described above.

Unless the context clearly requires otherwise, the expressions "comprise", "include", "contain", and the like should be understood to mean that they include the stated integer(s) or group of integers but are not excluding other integers or sets of integers.

Hereinafter, the present invention will be described in more detail with reference to exemplary embodiments. These exemplary embodiments are given for specifically illustrating the present invention, and the scope of the present invention is not limited to these exemplary embodiments.

### Example 1: Preparation of GLP-1 receptor (GLP-1R) antagonist analogs and acylated GLP-1 receptor (GLP-1R) antagonist analog conjugates

With respect to the synthesis of GLP-1R antagonist analogs, an automated peptide synthesizer (Symphony X, Gyros Protein Tech.) using solid-phase synthesis was employed. Rink amide resin was used for the amidation of the C-terminus. Respective amino acids were sequentially synthesized from the C-terminus to the N-terminus.

Fmoc (9H-fluoren-9-ylmethoxycarbonyl) protected amino acids (4 equivalents relative to peptide-resin), HOBt (1-hydroxybenzotriazole, 4 equivalents relative to peptide-resin), and DIC (diisopropylcarbodiimide, 8 equivalents relative to peptide-resin) were used for the sequential linkage of the amino acids.

With respect to acylated GLP-1R antagonist analog conjugates, peptides were synthesized by the above-described method using acylated amino acids K(1) to K(4).
[K(1)] C20diacid-γGlu-(AEEA)₂-Lys 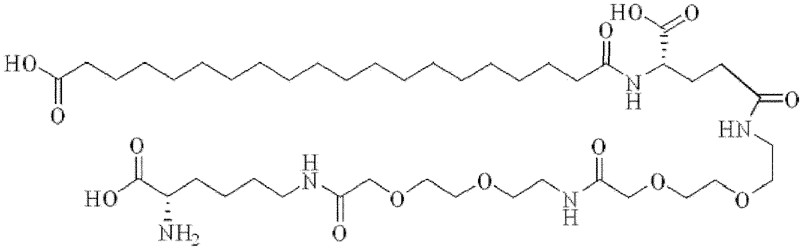
[K(2)] C18diacid-γGlu-(AEEA)₂-Lys 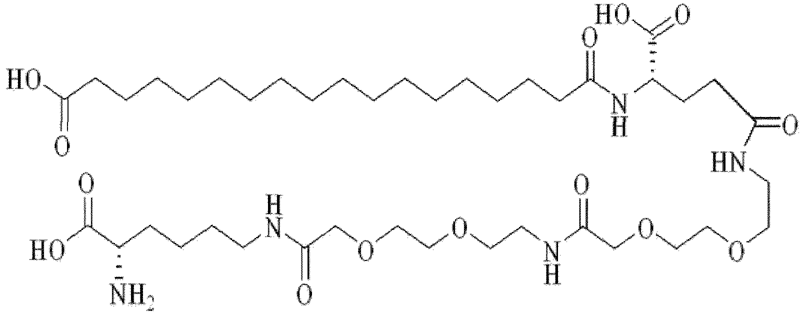
[K(3)] C16acid-γGlu-(AEEA)₂-Lys 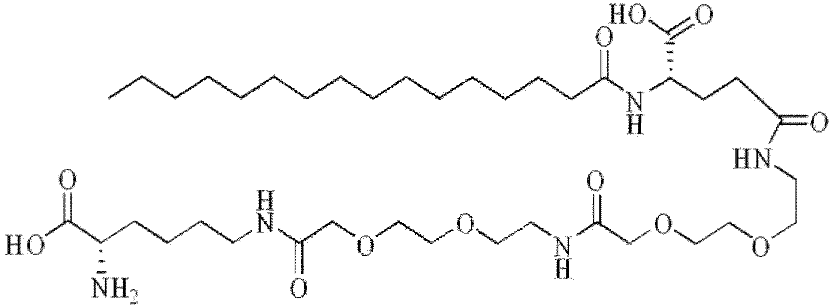
[K(4)] C16diacid-γGlu-(AEEA)₂-Lys

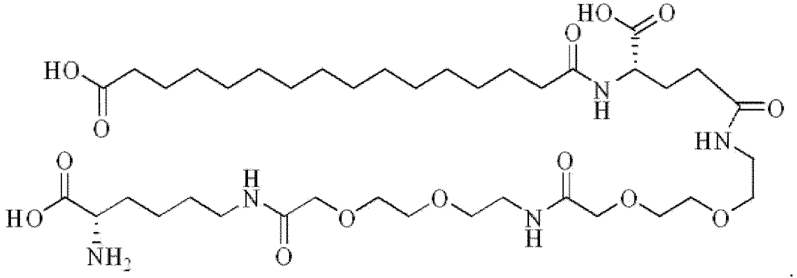

Fmoc protecting groups were removed by adding 8 mL of 20% piperidine/DMF (2 x 5 minutes) to the reaction vessel containing the resin in an automatic synthesizer. To remove remaining impurities, washing with 12 mL of DMF (6 x 10 seconds) was conducted after each step. After the completion of the synthesis, the N-terminus was acetylated with 6 mL of 34% acetic anhydride/DMF and 3.5 mL of 14% N,N-diisopropyl/DMF. When the peptides were cleaved from the resin, unremoved protecting groups were de-protected together.

The sequences of GLP-1R antagonist analogs and acylated GLP-1R antagonist analog conjugates prepared by such procedures are shown in Table 1 below.

**TABLE 1**

| SEQ ID NO | Amino acid sequence |
|---|---|
| 1 | TFTSDLSKQMEEEAVRLFIEWLKNGGPSSGAPPPS |
| 2 | TSDLSKQMEEEAVRLFIEWLKNGGPSSGAPPPS |
| 3 | DLSKQMEEEAVRLFIEWLKNGGPSSGAPPPS |
| 4 | DLSAQMEEEAVRLFIEWLKNGGPSSGAPPPS |
| 5 | DLSSQMEEEAVRLFIEWLKNGGPSSGAPPPS |
| 6 | QMEEEAVRLFIEWLKNGGPSSGAPPPS |
| 7 | TSDVSSYLEEEAVRLFIAWLVKGGPSSGAPPPS |
| 8 | DVSSYLEEEAVRLFIAWLVKGGPSSGAPPPS |
| 9 | DVSSYLEEQAVREFIAWLVKGGPSSGAPPPS |
| 10 | DLSSYLEEEAVRLFIEWLKNGGPSSGAPPPS |
| 11 | DVSSYLEEEAVRLFIEWLKNGGPSSGAPPPS |
| 12 | DVSSYLEEEAVRLFIAWLVKGGPSSGAPPPSC |
| 13 | DLSSYLEEEAVRLFIEWLK(1)NGGPSSGAPPPS |
| 14 | DLSSYLEEEAARLFIEWLK(2)NGGPSSGAPPPS |
| 15 | DVSSYLEEEAVRLFIAWLVK(2)GGPSSGAPPPS |
| 16 | DVSSYLEEEAARLFIAWLVK(2)GGPSSGAPPPS |
| 17 | DLSSYLEEEAVRLFIEWLKNGGPSSGAPPPSK(2) |
| 18 | DVSSYLEEEAVRLFIAWLVKGGPSSGAPPPSK(2) |
| 19 | DLSSYLEEEAVRLFIEWLKNGGPSSGAPPPSK(1) |
| 20 | DVSSYLEEEAVRLFIAWLVKGGPSSGAPPPSK(1) |
| 21 | DLSSYLEEEAVRLFIEWLKNGGPSSGAPPPSK(3) |
| 22 | DLSSYLEEEAVRLFIEWLKNGGPSSGAPPPSK(4) |

In Table 1, K(1) to K(4) indicate that amino acids at the corresponding sites are acylated amino acids with the above structures, respectively.

The synthesized GLP-1R antagonist analogs and acylated GLP-1R antagonist analog conjugates were purified by reverse-phase chromatography. The purity of the synthesized peptides was checked by analytical liquid chromatography (RP-HPLC), and a purity of 90% or more was determined to be a level suitable for use in tests. Additionally, the molecular weight and information of the peptides were identified using liquid chromatography/mass spectrometry (LC/MS).

The synthesized peptides were stored at -20°C until used for tests.

### Example 2: In vitro activities of GLP-1 receptor (GLP-1R) antagonist analogs and acylated GLP-1 receptor (GLP-1R) antagonist analog conjugates

To determine the activities of the GLP-1R antagonist analogs and acylated GLP-1R antagonist analog conjugates, the measurement of *in vitro* cell activity using cell lines transfected with human GLP-1R (hGLP-1R) was employed. Each of the cell lines was obtained by transfecting Chinese hamster ovary (CHO) cells to express human GLP-1R, and was suitable for the determination of the activities of GLP-1R antagonists.

To determine the activities of the GLP-1R antagonist analogs and acylated GLP-1R antagonist analog conjugates, human GLP-1 was diluted from 9000 pM to 0.15 pM by 3-fold dilutions. To identify antagonism, a comparative substance [exendin-3 (9-39) amide (Tocris Bioscience, UK, Cat No. 2081), SEQ ID NO: 24], GLP-1R antagonist analogs (SEQ ID NOs: 1 to 12), and acylated GLP-1R antagonist analog conjugates (SEQ ID NOs: 13 to 22) were serially diluted from 9000 pM to 0.15 pM by 3-fold dilutions on plates with 0.1 nM GLP-1 added thereto. The human GLP-1R-expressed CHO cells were cultured in a 384-well plate for 24 hours. Thereafter, the culture was removed, and 10 µL of each serially diluted substance was added to the plate, followed by incubation at room temperature for 30 minutes. Then, 5 µL of Eu-cAMP tracer was added, and then 5 µL of a buffer containing cAMP antibodies was added, followed by incubation at room temperature for 60 minutes. Upon completion of the reaction, the cell lysates were applied to a LANCE *Ultra* cAMP kit (PerkinElmer, USA), and the half-maximal inhibitory concentrations (IC₅₀) were calculated through the degrees of cAMP inhibition by GLP-1R antagonist analogs and acylated GLP-1R antagonist analog conjugates, followed by comparison with each other. As a result, the relative activities compared with the comparative substance are shown in Table 2 below.

**TABLE 2**

| Relative activity percents of GLP-1R analogs and acylated analog conjugates | |
|---|---|
| SEQ ID NO | *In vitro* activity compared with comparative substance (%) |
| Comparative substance (SEQ ID NO: 24) | 100 |
| SEQ ID NO: 1 | 69.1 |
| SEQ ID NO: 2 | 69.8 |
| SEQ ID NO: 3 | 134.7 |
| SEQ ID NO: 4 | 74.3 |
| SEQ ID NO: 5 | 85.0 |
| SEQ ID NO: 6 | 13.1 |
| SEQ ID NO: 7 | 195.5 |
| SEQ ID NO: 8 | 238.5 |
| SEQ ID NO: 9 | 188.6 |
| SEQ ID NO: 10 | 249.8 |
| SEQ ID NO: 11 | 166.1 |
| SEQ ID NO: 12 | 189.3 |
| SEQ ID NO: 13 | 4.8 |
| SEQ ID NO: 14 | 1.7 |
| SEQ ID NO: 15 | 14.3 |
| SEQ ID NO: 16 | 13.5 |
| SEQ ID NO: 17 | 91.8 |
| SEQ ID NO: 18 | 19.8 |
| SEQ ID NO: 19 | 33.6 |
| SEQ ID NO: 20 | 15.1 |
| SEQ ID NO: 21 | 380.1 |
| SEQ ID NO: 22 | 251.9 |

The test results confirmed that the GLP-1R antagonist analogs and the acylated GLP-1R antagonist analog conjugates prepared in Example 1 could act on human GLP-1 receptor (GLP-1R) to antagonize the GLP-1 receptor.

### Example 3: In vitro activities of acylated GLP-1 receptor (GLP-1R) antagonist analog conjugates

To determine the activities of the acylated GLP-1R antagonist analog conjugates, the measurement of *in vitro* cell activity using cell lines transfected with human (hGLP-1R) or mouse (mGLP-1R) GLP-1R was employed. Each of the cell lines was obtained by transfecting Chinese hamster ovary (CHO) cells to express human and mouse GLP-1R, separately, and was suitable for the determination of the activities of GLP-1R antagonists.

To determine the activities of the acylated GLP-1R antagonist analog conjugates, human GLP-1 was diluted from 9000 pM to 0.15 pM by 3-fold dilutions. To identify antagonism, a comparative substance [exendin-3 (9-39) amide (Tocris Bioscience, UK, Cat No. 2081), SEQ ID NO: 24] and acylated GLP-1R antagonist analog conjugates (SEQ ID NOs: 17, 19, 21, and 22) were serially diluted from 9000 pM to 0.15 pM by 3-fold dilutions on plates with 0.1 M GLP-1 added thereto.

The acylated GLP-1R antagonist analog conjugates of SEQ ID NOs: 17, 19, 21, and 22 used in the present example have structures of (i) to (iv) below, respectively.

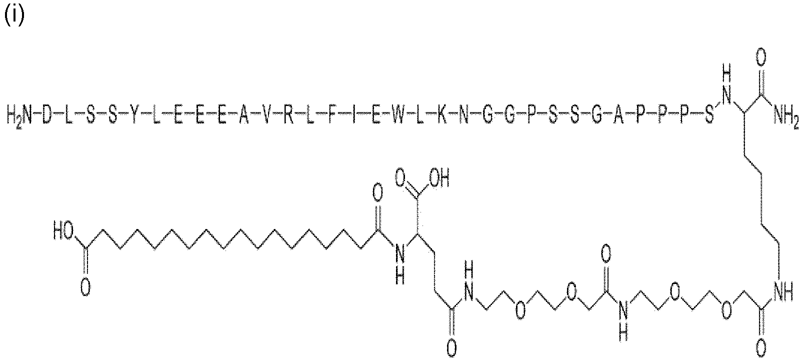

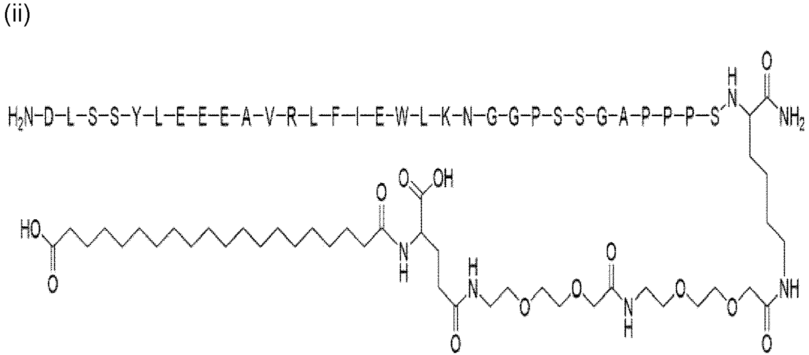

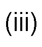

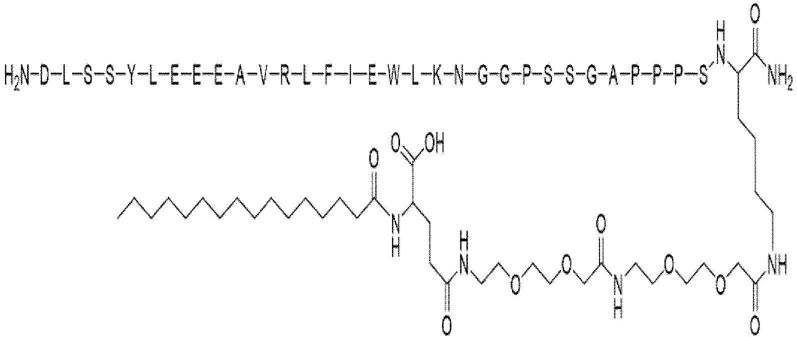

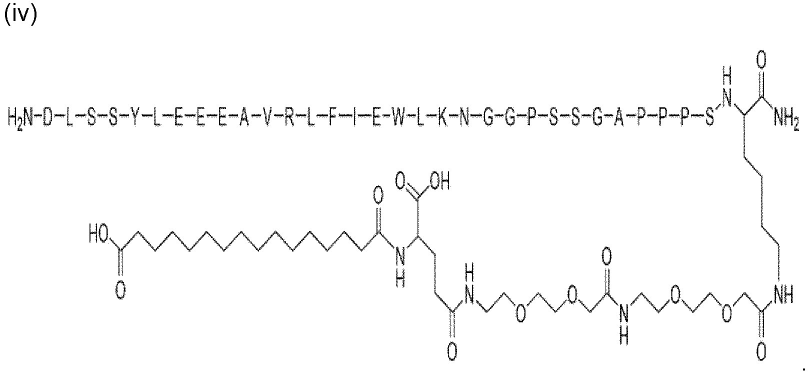

Additionally, to identify the effect of a fatty acid with binding affinity for albumin, the analog conjugates were diluted and prepared in a buffer containing 1% human serum albumin and mouse serum albumin. The human GLP-1R- or mouse GLP-1R-expressed CHO cells were cultured in a 384-well plate for 24 hours. Thereafter, the culture was removed, and 10 µL of each serially diluted substance was added to the plate, followed by incubation at room temperature for 30 minutes. Then, 5 µL of Eu-cAMP tracer was added, and then 5 µL of a buffer containing cAMP antibodies was added, followed by incubation at room temperature for 60 minutes. Upon completion of the reaction, the cell lysates were applied to LANCE *Ultra* cAMP kit (PerkinElmer, USA), and the half-maximal inhibitory concentrations (IC₅₀) were calculated through the degrees of cAMP inhibition by GLP-1R antagonist analogs and acylated GLP-1R antagonist analog conjugates, followed by comparison with each other. As a result, the relative activities compared with the comparative substance are shown in Table 3 below.

**TABLE 3**

| Relative activity proportions of acylated GLP-1R antagonist analog conjugates | | | | |
|---|---|---|---|---|
| | *In vitro* activity compared with comparative substance (%) | | | |
| | hGLP-1R / CHO cell line | | mGLP-1R / CHO cell line | |
| SEQ ID NO | Albumin-free | 1% human serum albumin | Albumin-free | 1% mouse serum albumin |
| Comparative substance (SEQ ID NO: 24) | 100 | 100 | 100 | 100 |
| SEQ ID NO: 17 | 91.8 | 4.3 | 50.1 | 9.2 |
| SEQ ID NO: 19 | 33.6 | 2.4 | 12.6 | 3.2 |
| SEQ ID NO: 21 | 380.1 | 29.4 | 95.7 | 27.0 |
| SEQ ID NO: 22 | 251.9 | 11.5 | 162.7 | 32.7 |

The GLP-1R antagonist analogs and the acylated GLP-1R antagonist analog conjugates prepared in Example 1 could act on both human and mouse GLP-1R receptors to antagonize the receptors, and especially, as explicitly stated in Table 3, in terms of potency, SEQ ID NOs: 21 and 22 having a C16 fatty acid exhibited excellent activity as antagonists compared with the comparative substance, followed by SEQ ID NO: 17 having a C18 fatty acid and then SEQ ID NO: 19 having a C20 fatty acid, among the acylated GLP-1R antagonist analog conjugates.

### Example 4: Blood glucose regulating effect of GLP-1 receptor (GLP-1R) antagonist analog conjugate (SEQ ID NO: 17)

To investigate the effect of the acylated GLP-1 receptor antagonist analog conjugate (SEQ ID NO: 17) on glucose tolerance, C57BL/6N mice were used as an animal model. The test was conducted on 8-week-old male mice, and during the study, five mice were housed in each group and had free access to drinking water. The lights were turned off from 6 PM to 6 AM.

The test groups are designated as Group 1: excipient (subcutaneous administration)-control grout (vehicle); Group 2: exendin-3 (9-39) 3690 µg/kg (1095.0 nmol/kg, subcutaneous administration), Group 3: GLP-1R antagonist analog conjugate (SEQ ID NO: 17) 3690 µg/kg (1061.9 nmol/kg, subcutaneous administration), and Group 4: GLP-1R antagonist analog conjugate (SEQ ID NO: 17) 7380 µg/kg (2123.8 nmol/kg, subcutaneous administration). The administration of the drugs was completed 24 hours before the oral glucose tolerance test (OGTT) for Groups 1, 3, and 4, and 30 minutes before for Group 2. Particularly, the dose was expressed as a value based on the value obtained by subtracting the mass of the fatty acid moieties from the total mass of the acylated GLP-1 receptor antagonist analog conjugate, that is, the value obtained by summing the mass of each of only polypeptide moieties.

As for the OGTT, all the groups were orally administered 2 g/kg glucose once after 4-hour fasting, and then blood samples were consecutively collected from the tail vein at specific time intervals (0, 15, 30, 60, and 120 minutes) to measure blood glucose levels by a OneTouch blood glucose meter (OneTouch Select^{®}, LifeScan, USA). Statistical analysis was performed using an unpaired t-test to compare between the control group and the test groups (# ~ ###p < 0.05 ~ 0.001).

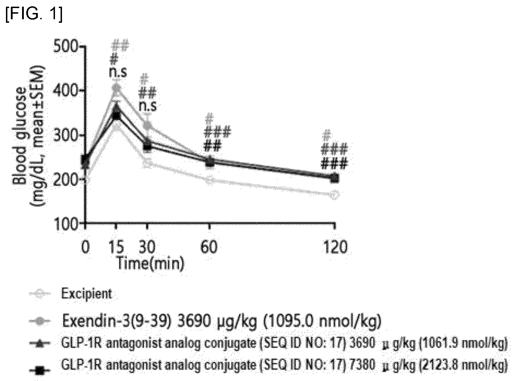

The results confirmed that the GLP-1R antagonist analog conjugate of SEQ ID NO: 17 increased the blood glucose levels *in vivo* compared with the control group at all the doses (FIG. 1). This indicates that the GLP-1R antagonist analog or acylated GLP-1R antagonist analog conjugate according to the present invention can exhibit an effect in the treatment of congenital hyperinsulinemia through the blood glucose regulating ability thereof.

### Example 5: Blood glucose regulating effect of GLP-1 receptor (GLP-1R) antagonist analog conjugate (SEQ ID NO: 17) in postbariatric hypoglycemia (PBH) rat model induced by vertical sleeve gastrectomy (VSG)

A PBH rat model was established by performing sham operations or VSG surgery on SD rats. The rats were fed a high-fat diet (HFD) for 19 weeks, and divided randomly into two groups with 10 animals each (an excipient control group and a GLP-1R antagonist analog conjugate administered group). A mixed meal tolerance test (MMTT) was conducted 4 hours after subcutaneous administration of the excipient and the GLP-1R antagonist analog conjugate (SEQ ID NO: 17) at 3720 µg/kg (1070.5 nmol/kg). As for the MMTT, all the groups were orally administered 2 mL of a mixed meal (Ensure Plus^{®}, Abbott, USA) once after 5-hour fasting, and then blood samples were consecutively collected at specific time intervals (0, 15, 30, 45, 60, and 120 minutes) to measure blood glucose levels (GDoctor, Allmedicus Co., Korea). Statistical analysis was performed using the one-way ANOVA to compare between the VSG rat excipient group (control group) and the test groups (* ~ ***p < 0.05 ~ 0.001). Particularly, the dose was expressed as a value based on the value obtained by subtracting the mass of the fatty acid moieties from the total mass of the acylated GLP-1 receptor antagonist analog conjugate, that is, the value obtained by summing the masses of each of only polypeptide moieties.

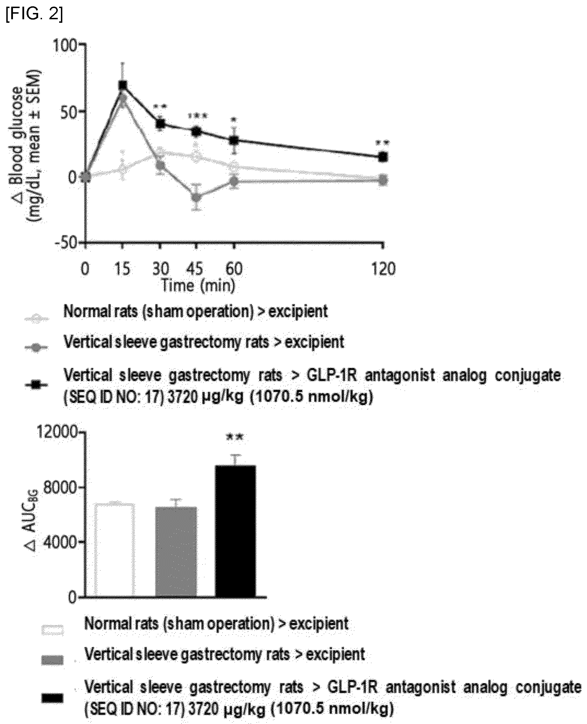

The results confirmed that the GLP-1R antagonist analog conjugate of SEQ ID NO: 17 significantly increased blood glucose levels *in vivo* compared with PBH rats administered the excipient (FIG. 2).

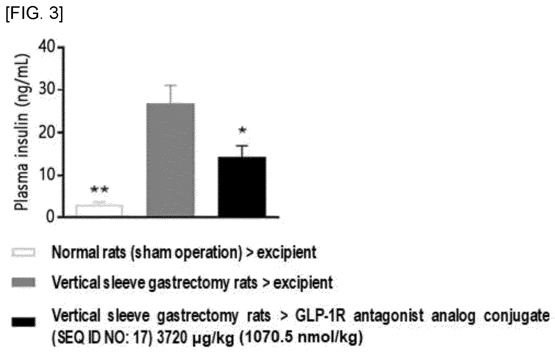

Additionally, 15 minutes after the MMTT test, plasma was isolated from blood samples, and the insulin secretion level was measured using a rat insulin ELISA kit (Multi species GLP-1 total ELISA, Merck Millipore, USA). The results confirmed that the GLP-1R antagonist analog conjugate of SEQ ID NO: 17 showed a 47% reduction in the secretion of insulin compared with the excipient control group (FIG. 3). This indicates that the GLP-1R antagonist analog or acylated GLP-1R antagonist analog conjugate according to the present invention can exhibit an effect in the treatment of hypoglycemia, especially, postbariatric hypoglycemia, through the blood glucose regulating ability thereof.

It was confirmed from the above examples that the GLP-1 receptor antagonist analogs and acylated GLP-1 receptor antagonist analog conjugates prepared in the present invention can not only serve as antagonists against the GLP-1 receptor, but can also be used, on the basis of such activities, as a useful therapeutic agent for congenital hyperinsulinemia, hypoglycemia, especially, postbariatric hypoglycemia.

While the present invention has been described with reference to the particular illustrative embodiments, a person skilled in the art to which the present invention pertains can understand that the present invention may be embodied in other specific forms without departing from the technical spirit or essential characteristics thereof. Therefore, the exemplary embodiments described above should be construed as being exemplified and not limiting the present disclosure. The scope of the invention should be construed that the meaning and scope of the appended claims rather than the detailed description and all changes or variations derived from the equivalent concepts fall within the scope of the present invention.

## Claims

1. A GLP-1 receptor antagonist analog represented by General Formula 1 below:
X1-X2-X3-X4-X5-X6-X7-X8-X9-X10-E-E-X13-A-X15-R-X17-F-I-X20-W-L-X23-X24-G-G-P-S-S-G-A-P-P-P-S-X36 (General Formula 1, SEQ ID NO: 25) wherein, in General Formula 1,
X1 is threonine or absent;
X2 is phenylalanine or absent;
X3 is threonine or absent;
X4 is serine or absent;
X5 is aspartic acid or absent;
X6 is leucine, valine, or absent;
X7 is serine or absent;
X8 is alanine, lysine, serine, or absent;
X9 is glutamine or tyrosine;
X10 is methionine or leucine;
X13 is glutamic acid or glutamine;
X15 is alanine or valine;
X17 is leucine or glutamic acid;
X20 is glutamic acid or alanine;
X23 is lysine, valine, or an acylated amino acid;
X24 is asparagine, lysine, or an acylated amino acid;
X36 is cysteine, lysine, an acylated amino acid, or absent; and
the symbol "-" represents a peptide bond.

2. The GLP-1 receptor antagonist analog of claim 1, wherein, in General Formula 1,
X5 is aspartic acid;
X6 is leucine or valine;
X7 is serine;
X8 is lysine or serine;
X15 is valine;
X23 is lysine or valine;
X24 is asparagine or lysine.

3. The GLP-1 receptor antagonist analog of claim 1, wherein, in General Formula 1,
X1 to X4 are absent;
X5 is aspartic acid;
X6 is leucine;
X7 is serine;
X8 is serine;
X9 is tyrosine;
X10 is leucine;
X13 is glutamic acid;
X15 is valine;
X17 is leucine;
X20 is glutamic acid;
X23 is lysine;
X24 is asparagine; and
X36 is lysine or an acylated amino acid.

4. The GLP-1 receptor antagonist analog of claim 1, wherein the acylated amino acid is any one of amino acids represented by K(1) to K(4):
[K(1)] C20diacid-γGlu-(AEEA)₂-Lys; 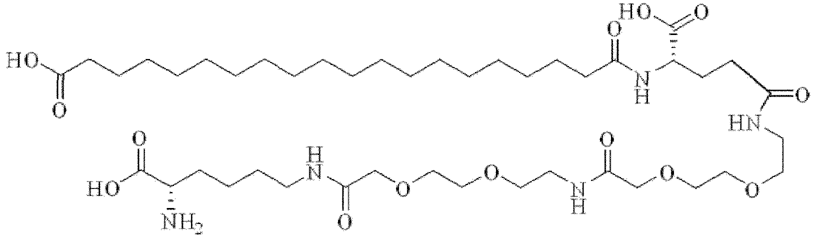
[K(2)] C18diacid-γGlu-(AEEA)₂-Lys; 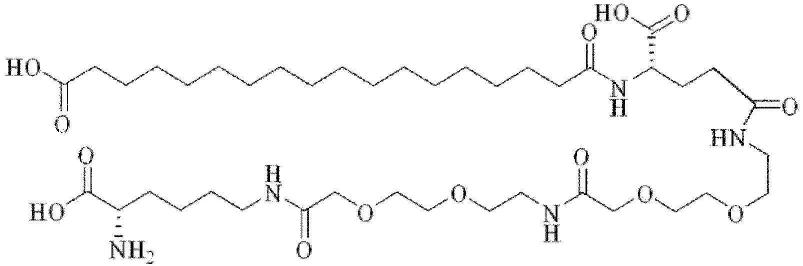
[K(3)] C16acid-γGlu-(AEEA)₂-Lys; and 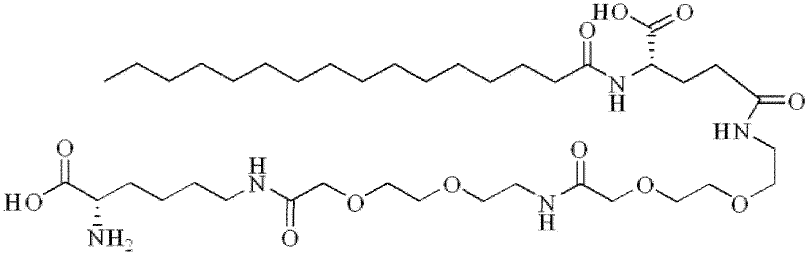
[K(4)] C16diacid-γGlu-(AEEA)₂-Lys.

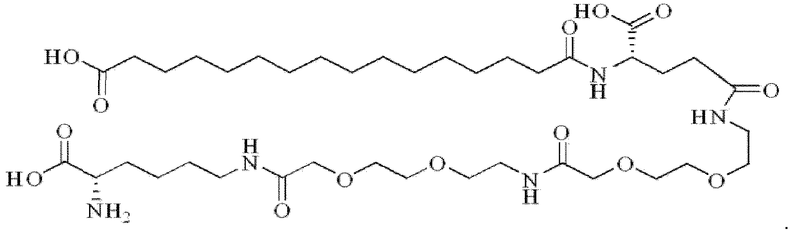

5. The GLP-1 receptor antagonist analog of claim 1, wherein an acyl group is attached to at least one amino acid of the GLP-1 receptor antagonist analog via a linker containing (2-(2-aminoethoxy)ethoxy)acetic acid (AEEA).

6. The GLP-1 receptor antagonist analog of claim 1, wherein the C-terminus of the GLP-1 receptor antagonist analog is amidated.

7. The GLP-1 receptor antagonist analog of claim 1, wherein the GLP-1 receptor antagonist analog is acylated with a C1 to C30 straight or branched chain acyl group containing one or two carboxylic acids.

8. The GLP-1 receptor antagonist analog of claim 7, wherein the acyl group is a C4 to C30 fatty acid or dicarboxylic acid.

9. The GLP-1 receptor antagonist analog of claim 7, wherein an amino acid located at the C-terminus or lysine residue of the GLP-1 receptor antagonist analog is acylated.

10. The GLP-1 receptor antagonist analog of claim 7, wherein the C-terminus of the GLP-1 receptor antagonist analog is amidated.

11. The GLP-1 receptor antagonist analog of claim 1, wherein the GLP-1 receptor antagonist analog includes any one sequence selected from the group consisting of the amino acid sequences of SEQ ID NOs: 1 to 22.

12. The GLP-1 receptor antagonist analog of claim 1, wherein the GLP-1 receptor antagonist analog includes any one sequence selected from the group consisting of the amino acid sequences of SEQ ID NOs: 3, 7 to 12, 21, and 22.

13. The GLP-1 receptor antagonist analog of claim 1, wherein the GLP-1 receptor antagonist analog includes any one sequence selected from the group consisting of the amino acid sequences of SEQ ID NOs: 17, 19, 21, and 22.

14. The GLP-1 receptor antagonist analog of claim 1, wherein the GLP-1 receptor antagonist analog has any one structure of (i) to (iv) below:

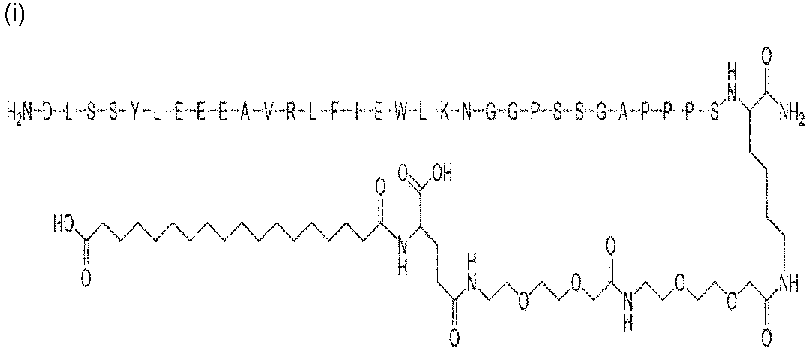

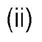

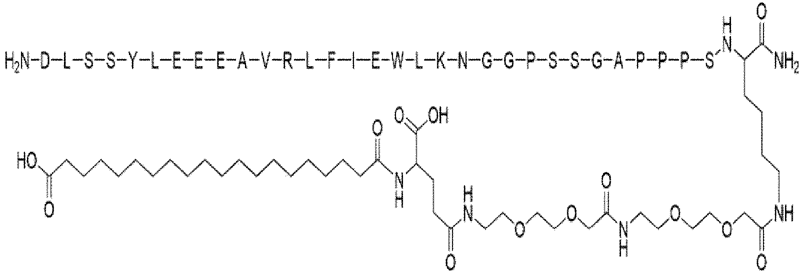

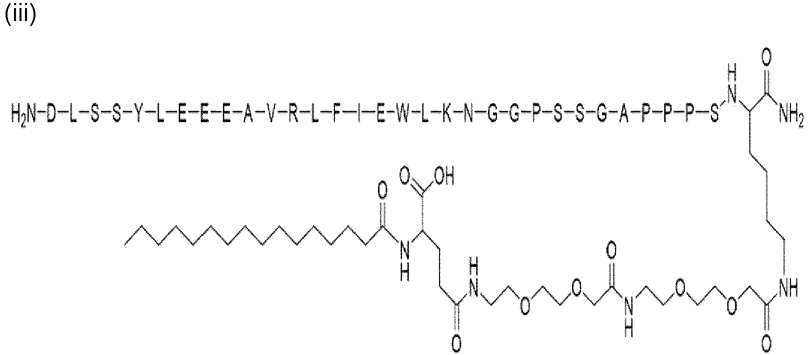

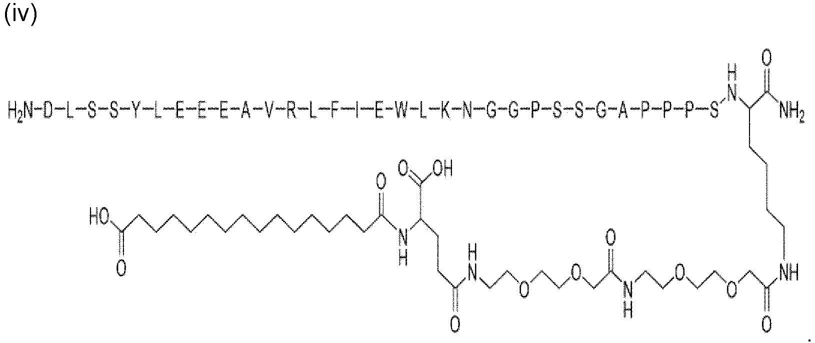

15. A pharmaceutical composition for preventing or treating congenital hyperinsulinemia or hypoglycemia containing the GLP-1 receptor antagonist analog of any one of claims 1 to 14.

16. The pharmaceutical composition of claim 15, wherein the hypoglycemia is postbariatric hypoglycemia (PBH).

17. The pharmaceutical composition of claim 16, wherein the postbariatric hypoglycemia is caused by bariatric surgery.
